# EUROPEAN PATENT APPLICATION

(11) **EP 4 733 748 A1**
(43) Date of publication of application: **29.04.2026**
(21) Application number: 24826130.7
(22) Date of filing: 17.05.2024
(51) Int. Cl.: G01N 21/84, G01N 33/22, G01N 1/28, G01N 1/38, G06N 3/08, G06T 7/00, G01N 21/17, G06V 10/82, G06V 20/69

(54) **MATERIAL ANALYSIS METHOD**

(30) Priority: 23.06.2023 KR 20230081284
(71) Applicant: Hyundai Steel Company, Incheon 22525 (KR)
(72) Inventor: PARK, Tae Chang, Incheon 22525 (KR); KIM, Nam Uk, Incheon 22525 (KR); LEE, Jong Hyup, Incheon 22525 (KR); KIM, Byong Chul, Incheon 22525 (KR)
(74) Representative: Viering, Jentschura & Partner mbB Patent- und Rechtsanwälte
(86) International application number: PCT/KR2024/006698
(87) International publication number: WO 2024/262808

(57) **Abstract**

The present invention relates to a material analysis method which may comprise the steps of: (a) preparing a specimen for structure analysis; (b) training an artificial intelligence model with training images labelled with structural aspects of arbitrary specimens; (c) analyzing a plurality of images for analysis not labelled with structural aspects of specimens by using the trained artificial intelligence model, and removing images for analysis classified as preset noise structural aspects; and (d) using the trained artificial intelligence model to analyze the images for analysis that remain after the images for analysis classified as the noise structural aspects are removed, and classifying the remaining images for analysis as at least one target structural aspect.

## Description

### TECHNICAL FIELD

The present invention relates to a material analysis method, and more particularly, to a method capable of analyzing a microstructure of a material utilizing artificial intelligence.

### BACKGROUND ART

In the coking process of steelworks, coal is dry-distilled at a high temperature of 1,000 to 1,300°C to produce coke suitable for use in a blast furnace. Coal used as a raw material for coke is a mineral composed of carbonaceous material, and is composed of a mixture of multiple types of Macerals, which are microscopic components.

Maceral is an organic component distinguished by size and shape mainly using a microscope, and is used as a minimum unit for identification when classifying coal microstructure. The classification criteria for Maceral following the KS standard (KS E ISO 7404-3) depend on visual analysis (point counting method) by an examiner, and require counting at least 500 points, which not only causes analysis variation among examiners but also has a problem of taking a long time.

### DETAILED DESCRIPTION OF THE INVENTION

### TECHNICAL PROBLEM

The present invention is to solve various problems including the above problems, and aims to provide a material analysis method that automates the visual analysis of a small number of skilled workers for distinguishing coal Maceral and calculating proportion by using an artificial intelligence (AI) model based on deep learning, so that even unskilled general workers can easily and quickly distinguish coal Maceral and calculate proportion. However, these problems are exemplary and do not limit the scope of the present invention.

### TECHNICAL SOLUTION

According to an embodiment of the present invention, a material analysis method is provided. The material analysis method may include: (a) preparing a specimen for analyzing a microstructure; (b) acquiring a plurality of specimen images by photographing an inspection region set on one surface of the specimen; (c) preprocessing the plurality of specimen images to obtain a plurality of analytical images; and (d) analyzing the plurality of analytical images using a trained microstructure classification artificial intelligence (AI) model to classify into at least one target microstructure pattern.

According to an embodiment of the present invention, the step (a) may include: (a-1) crushing a material into particles having a predetermined size; and (a-2) mixing the particles with a binder to fabricate the specimen.

According to an embodiment of the present invention, the material may be coal, and the binder may be epoxy.

According to an embodiment of the present invention, the step (c) may be a step of obtaining an analytical image by extracting an attention region that the Al model sets for analysis on the specimen image utilizing a Class Activation Map (CAM).

According to an embodiment of the present invention, the attention region may be a region corresponding to a central region on the specimen image.

According to an embodiment of the present invention, the step (c) may be a step of analyzing a plurality of specimen images in which the microstructure pattern of the specimen is not labeled using a trained noise removal artificial intelligence (AI) model to remove analytical images classified as a preset noise microstructure pattern.

According to an embodiment of the present invention, the noise microstructure pattern may be an image in which the microstructure pattern of the specimen is classified as a binder by the noise removal Al model.

According to an embodiment of the present invention, the noise removal Al model may be an Al model using a Residual neural network (ResNet) or MobileNet based deep learning network.

According to an embodiment of the present invention, in the step (d), the microstructure classification Al model may be an Al model using an Inception based deep learning network.

According to an embodiment of the present invention, before the step (c), the method may further include: (f) training a noise removal Al model or a microstructure classification Al model with training images in which the microstructure pattern of an arbitrary specimen is labeled.

According to an embodiment of the present invention, in the step (d), the target microstructure pattern may be an image in which the microstructure pattern of the specimen is classified into at least one of Vitrinite, Exinite, Fusinite, Semi-fusinite, Mineral, and combinations thereof by the second Al model.

According to an embodiment of the present invention, after the step (d), the method may further include: (e) calculating a proportion of the classified microstructure patterns of the specimen.

### ADVANTAGEOUS EFFECTS

According to an embodiment of the present invention made as described above, by setting a hexagonal inspection region, characteristics of the entire circular cross-section can be sufficiently reflected and efficient analysis progress time can be secured, and there is an effect that Maceral microstructure, which is difficult for general workers to distinguish, can be distinguished easily and quickly using artificial intelligence.

In addition, by independently using an Al model for classifying epoxy images and an Al model for classifying Maceral microstructure, there is an effect that Maceral microstructure can be classified with higher precision, and accuracy can be increased by extracting a central region of the specimen image and proceeding with analysis. Of course, the scope of the present invention is not limited by these effects.

### DESCRIPTION OF THE DRAWINGS

FIG. 1 is a flowchart sequentially showing each step of a material analysis method according to an embodiment of the present invention.
FIG. 2 is a flowchart showing each step of a material analysis method according to an embodiment of the present invention in more detail.
FIG. 3 is an image schematically showing a structure of a noise removal artificial intelligence (AI) model according to an embodiment of the present invention.
FIG. 4 is an image schematically showing a structure of a microstructure classification Al model according to an embodiment of the present invention.
FIG. 5 is a table and image showing a confusion matrix and Maceral structure according to an embodiment of the present invention.
FIG. 6 is a table showing precision by extracting a central region of a specimen and inputting it to each microstructure classification Al model.
FIG. 7 is confusion matrix images showing an improvement degree of test precision when a central region of a specimen is extracted.
FIG. 8 is an image showing an attention region that an Al model sets for analysis on the specimen image.
FIG. 9 is a table showing accuracy by initial learning rate of noise removal Al models.
FIG. 10 is a table showing accuracy by initial learning rate of microstructure classification Al models.
FIG. 11 is a conceptual diagram schematically showing a material analysis apparatus according to an embodiment of the present invention.
FIG. 12 is an image exemplarily showing a hexagonal inspection region according to an embodiment of the present invention.

### MODE OF THE INVENTION

Hereinafter, various preferred embodiments of the present invention will be described in detail with reference to the accompanying drawings.

The embodiments of the present invention are provided to more completely explain the present invention to those skilled in the art, and the following embodiments may be modified in various different forms, and the scope of the present invention is not limited to the following embodiments. Rather, these embodiments are provided to make the present disclosure more faithful and complete, and to completely convey the spirit of the present invention to those skilled in the art. In addition, the thickness or size of each layer in the drawings is exaggerated for convenience and clarity of explanation.

Hereinafter, embodiments of the present invention will be described with reference to drawings schematically showing ideal embodiments of the present invention. In the drawings, for example, deformations of the illustrated shapes may be expected depending on manufacturing technology and tolerance. Therefore, embodiments of the spirit of the present invention should not be construed as being limited to the specific shapes of the regions shown in this specification, and should include, for example, changes in shape caused by manufacturing.

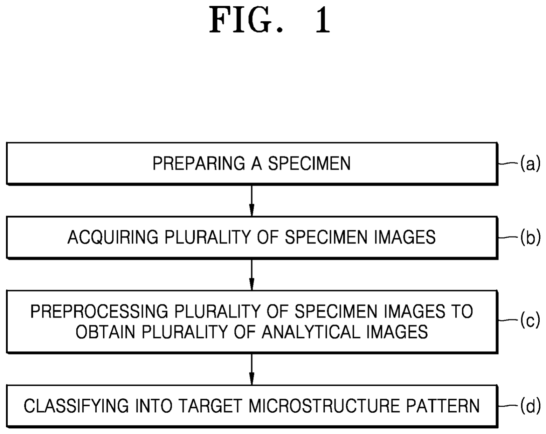

FIG. 1 is a flowchart sequentially showing each step of a material analysis method according to an embodiment of the present invention.

As shown in FIG. 1, the material analysis method according to an embodiment of the present invention may include (a) preparing a specimen for analyzing a microstructure, (b) acquiring a plurality of specimen images by photographing an inspection region set on one surface of the specimen, (c) preprocessing the plurality of specimen images to obtain a plurality of analytical images, and (d) analyzing the plurality of analytical images using a trained microstructure classification artificial intelligence (AI) model to classify into at least one target microstructure pattern.

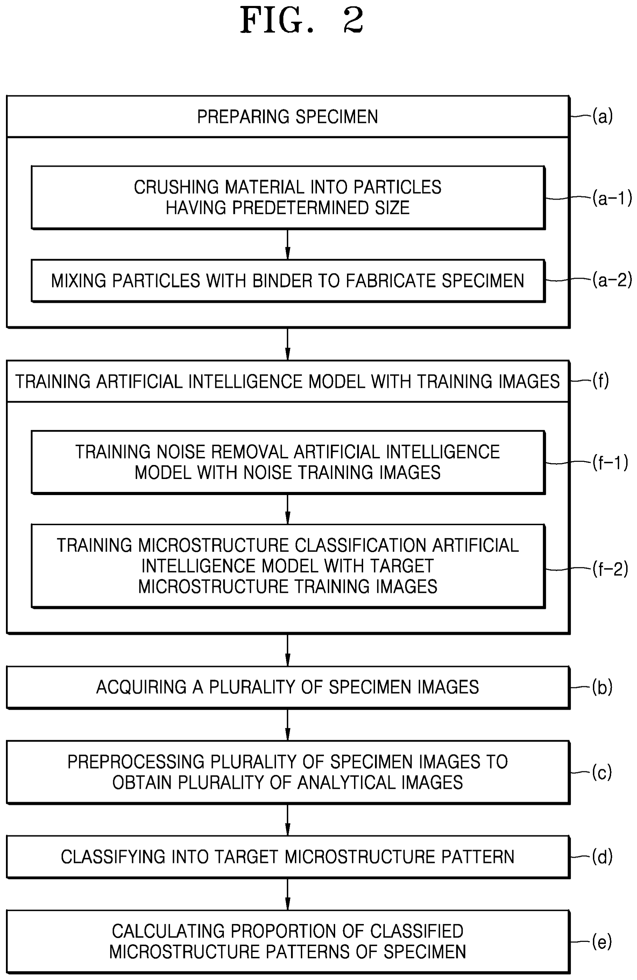

More specifically, as shown in FIG. 2, the step (a) of preparing a specimen for analyzing a microstructure may include (a-1) crushing a material into particles having a predetermined size, and (a-2) mixing the particles with a binder to fabricate the specimen.

At this time, the material is coal and the binder is epoxy, and a step of crushing coal into particles having a predetermined size, mixing the binder, and then fabricating into a specimen having an overall cylindrical shape by pressure molding may be performed.

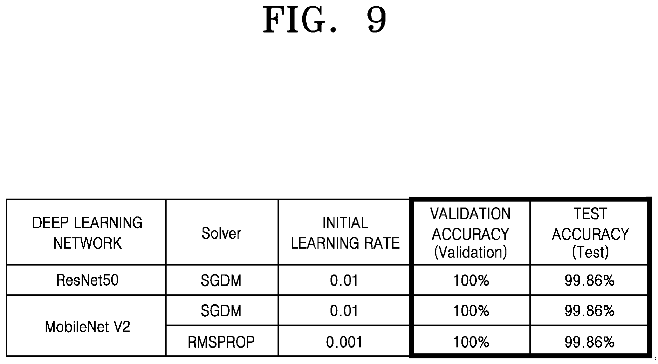

Subsequently, in the step (b) of acquiring a plurality of images for analysis by photographing an inspection region set on one surface of the specimen, the inspection region may be a region in which a plurality of inspection points are set to form an overall hexagonal shape. Such an inspection region may be a region in which a plurality of inspection points are set to form an overall hexagonal shape as shown in FIG. 9 to be described later.

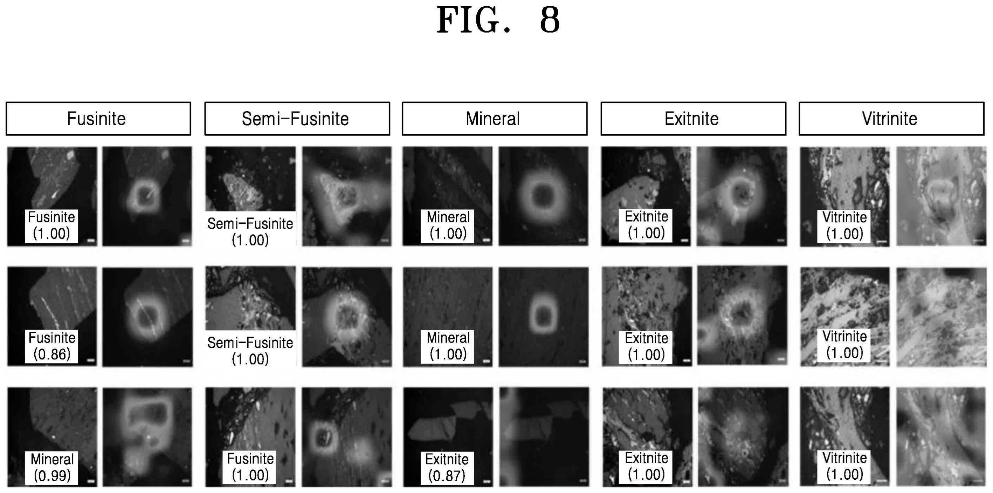

In addition, the step (c) of preprocessing the plurality of specimen images to obtain a plurality of analytical images may include extracting an attention region that the Al model sets for analysis on the specimen image utilizing a Class Activation Map (CAM) to obtain an analytical image. The attention region will be described in detail with reference to FIG. 8 to be described later.

In addition, the step (c) of preprocessing the plurality of specimen images to obtain a plurality of analytical images may include analyzing a plurality of specimen images in which the microstructure pattern of the specimen is not labeled using a trained noise removal Al model to remove analytical images classified as a preset noise microstructure pattern. The noise microstructure pattern may be an image in which the microstructure pattern of the specimen is classified as a binder, that is, epoxy, by the noise removal Al model.

At this time, more specifically, the noise removal Al model may determine and classify an image in which the microstructure pattern of the specimen is classified as a binder as a noise microstructure pattern, and the microstructure classification Al model may determine and classify an image in which the microstructure pattern of the specimen is classified into at least one of Vitrinite, Exinite, Fusinite, Semi-fusinite, Mineral, and combinations thereof as a target microstructure pattern.

In addition, as shown in FIG. 2, the material analysis method according to an embodiment of the present invention may further include, before the step (c) of removing images classified as a noise microstructure pattern, (f) training an Al model with training images in which the microstructure pattern of an arbitrary specimen is labeled.

Steps (a) to (e) shown in FIG. 2 may not necessarily be performed in order. For example, after training an Al model with an arbitrary specimen in step (f), a specimen for analyzing a microstructure may be prepared in step (a).

More specifically, the step (f) of training an Al model with training images may include (f-1) training a noise removal Al model with noise training images, and (f-2) training a microstructure classification Al model with target microstructure training images.

At this time, in the step (f-1) of training a noise removal Al model with noise training images, the noise training images may be images labeled by classifying the microstructure pattern of the specimen as a binder.

In addition, in the step (f-2) of training a microstructure classification Al model with target microstructure training images, the target microstructure training images may be images labeled by classifying the microstructure pattern of the specimen into at least one of Vitrinite, Exinite, Fusinite, Semi-fusinite, Mineral, and combinations thereof.

Hereinafter, steps (c) to (d) of the material analysis method according to an embodiment of the present invention will be described in detail.

According to an embodiment of the present invention, in step (c), an analytical image may be obtained by extracting an attention region that the Al model sets for analysis on the specimen image utilizing a Class Activation Map (CAM).

According to an embodiment of the present invention, in step (c), by removing in advance images classified as a preset noise microstructure pattern among a plurality of analytical images of the specimen using a noise removal Al model, the number of analytical images to be classified in step (d) can be reduced, and the remaining analytical images can be efficiently classified into a target microstructure pattern using a microstructure classification Al model.

In step (c) of the material analysis method according to an embodiment of the present invention, the noise removal Al model may be an Al model using a Residual neural network (ResNet) or MobileNet based deep learning network.

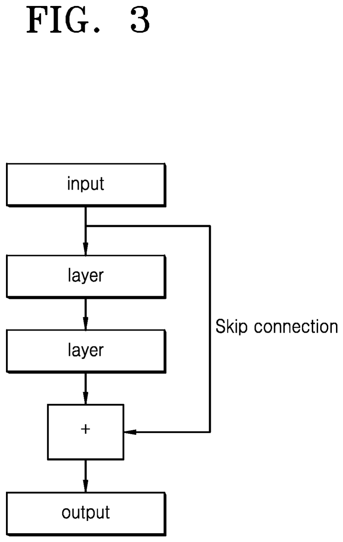

As shown in FIG. 3, the ResNet AI model can solve the information loss problem that occurs as the depth of the neural network increases by introducing a skip connection to calculate and learn the amount of change in each layer so that the difference between the previous layer and the next layer can be learned.

In addition, according to an embodiment of the present invention, through the skip connection structure of ResNet, features of epoxy images, which show a relatively simple form compared to complex Maceral microstructure, are effectively extracted and concatenated to the next layer, and even if the depth of each layer deepens, the extracted features are not blurred, so epoxy images can be classified with high precision.

In addition, although not shown in the drawings, MobileNet, designed to analyze images in real time on mobile devices, is an Al model that maintains a small model size and low computational cost while ensuring high accuracy, and can quickly and accurately classify only epoxy images.

In step (d) of the material analysis method according to an embodiment of the present invention, the microstructure classification Al model may be an Al model using an Inception based deep learning network. More specifically, it may be an Al model using an Inception V3 based deep learning network.

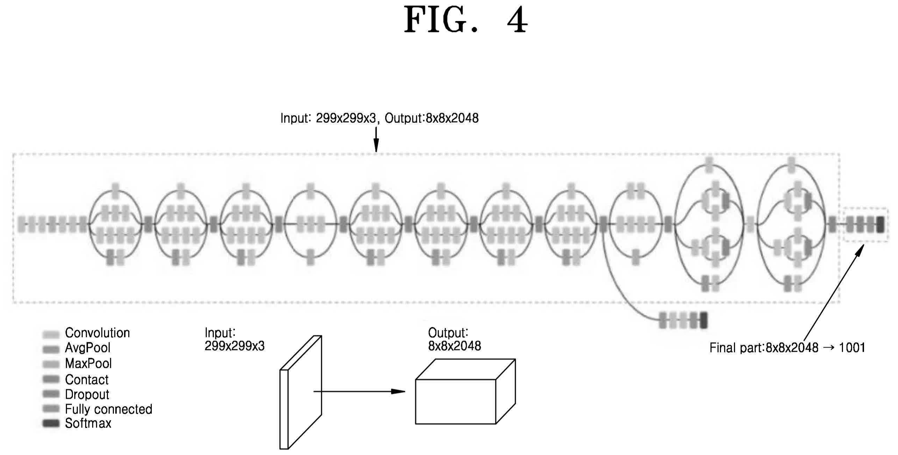

As shown in FIG. 4, the Inception V3 Al model may include an Inception module composed of a combination of a convolution layer, an average pooling layer, a max pooling layer, concatenation, Dropout, and Softmax. At this time, each Inception module can reduce the computational cost by calculating convolution layers divided into sizes such as 1×1, 3×3, and 5×5, and extract features of the input image in multiple stages.

In addition, according to an embodiment of the present invention, through the divided convolution layers, which are features of the Inception structure, more diverse characteristics can be found compared to an Al model having a single layer, so that coal Maceral microstructure images containing various microstructure patterns can be effectively classified.

Accordingly, when analyzing specimen images using a single Al model, the precision of distinguishing epoxy was in the 40% range and did not satisfy the required precision, but by independently using a noise removal Al model for classifying epoxy images and a microstructure classification Al model for classifying Maceral microstructure, epoxy could be distinguished with high precision in the 99% range, and Maceral microstructure could be classified with precision in the 94% range.

In addition, step (f) of the material analysis method according to an embodiment of the present invention may further include (f-3) verifying classification accuracy of a pre-trained Al model using a confusion matrix. More specifically, step (f-3) may include verifying classification accuracy of a noise removal Al model using a confusion matrix, and verifying classification accuracy of a microstructure classification Al model.

The confusion matrix can visualize results for predictions of the Al model and classification data by displaying them as a matrix. For example, the confusion matrix is a 2×2 matrix that combines true and false of predicted values and true and false of actual values to obtain a combination of true positive (TP), where the actual positive value is the same as the predicted positive, false positive (FP), where a negative value is incorrectly predicted as positive, true negative (TN), where the actual negative value and predicted negative value are the same, and false negative (FN), where a positive value is incorrectly predicted as negative.

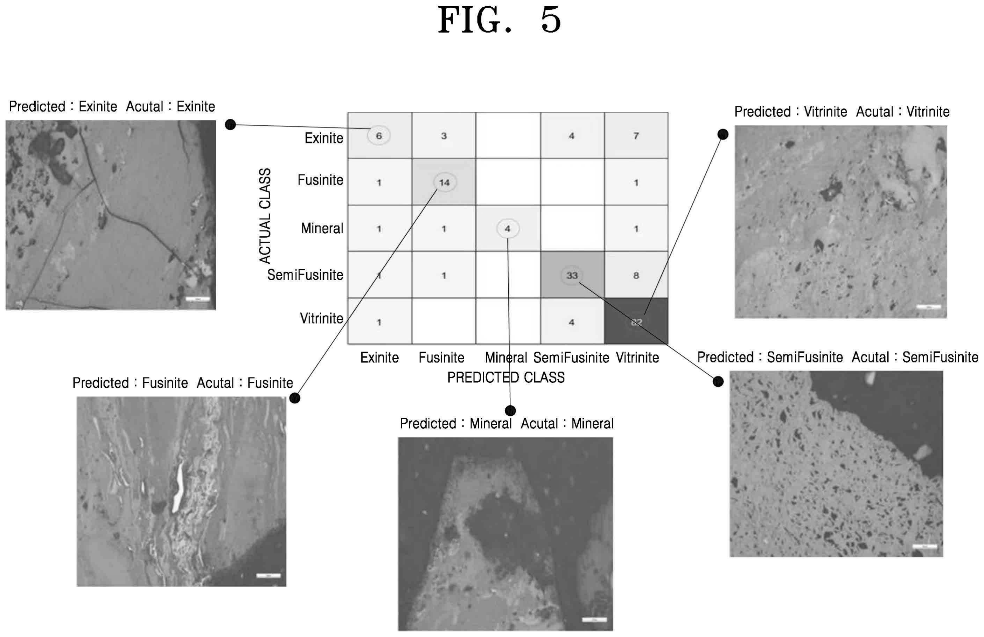

At this time, the confusion matrix according to an embodiment of the present invention used a 5×5 matrix by expanding the above-mentioned 2×2 confusion matrix, as shown in FIG. 5, so that Maceral, a microscopic component of coal, can be classified into five categories. In this case, true positive values where the actual positive value is the same as the predicted positive exist along a diagonal direction from the upper left to the lower right, and the remaining values may be a combination of false positive or false negative.

As shown in FIG. 5, the composition of Maceral in coal can be classified into Vitrinite, Exinite, Fusinite, Semi-fusinite, and Mineral.

More specifically, Vitrinite may constitute the majority of coal microstructures that exhibit caking property. Vitrinite is derived from woody tissue of plants and exhibits a smooth and homogeneous tissue surface. Exinite, which is used for controlling caking property, may be a tissue derived from the cuticles of plant leaves or small branches. Exinite has high volatile matter and tar content and exhibits a generally dark brown serrated shape. Inertinite may be a tissue having a three-dimensional configuration as an inert component that does not soften or melt. The major types of Inertinite include Fusinite, Semi-fusinite, and others. Mineral may be a small amount of mineral matter contained in coal, exhibiting a generally dark brown appearance.

In the case of conventional visual analysis, it was very difficult for general workers to distinguish Maceral, so they relied on the know-how of highly skilled workers. However, when applying the material analysis method according to an embodiment of the present invention, Maceral microstructure, which is difficult for general workers to distinguish, can be distinguished easily and quickly. For example, it is possible to easily distinguish Semi-fusinite, which has mixed characteristics of Vitrinite and Fusinite, and also, for example, it is possible to easily distinguish Exinite and Mineral, which are dark brown.

In addition, the step (d) of classifying analytical images into a target microstructure pattern may classify the remaining analytical images after removing epoxy images as a noise microstructure pattern in step (c) into at least one of the above-mentioned Vitrinite, Exinite, Fusinite, Semi-fusinite, Mineral, and combinations thereof.

In addition, the material analysis method according to an embodiment of the present invention may further include, after step (d), (e) calculating a proportion of the classified microstructure patterns of the specimen. For example, the Maceral microstructure of the coal sample to be analyzed can be calculated as a percentage (%) of Vitrinite, Exinite, Fusinite, Semi-fusinite, and Mineral with respect to the total volume.

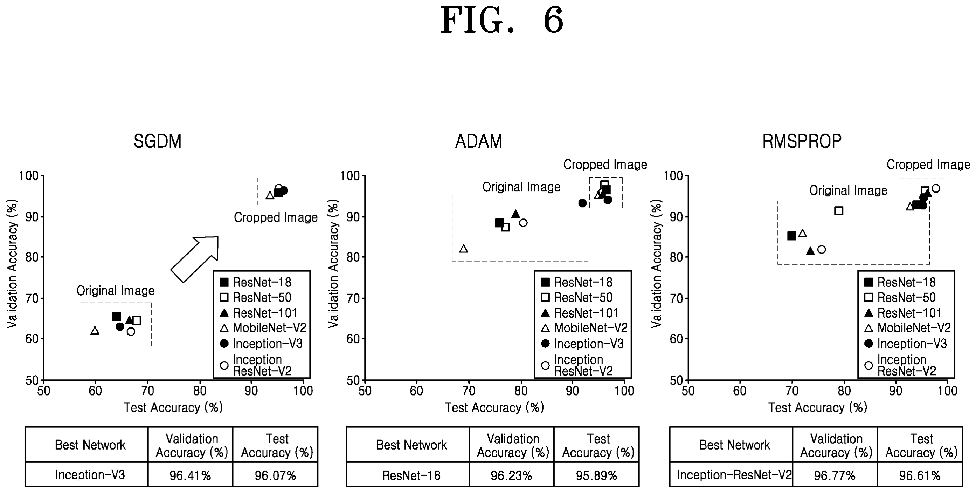

FIG. 6 is a table comparing results of extracting an attention region, which is a region corresponding to a central region on the specimen image, in step (c) of the material analysis method according to an embodiment of the present invention and inputting it to a Stochastic Gradient Descent with Momentum (SGDM), Adaptive Moment Estimation (ADAM), or Root Mean Square Propagation (RMSPROP) solver (or optimizer), with results of inputting the existing entire specimen image.

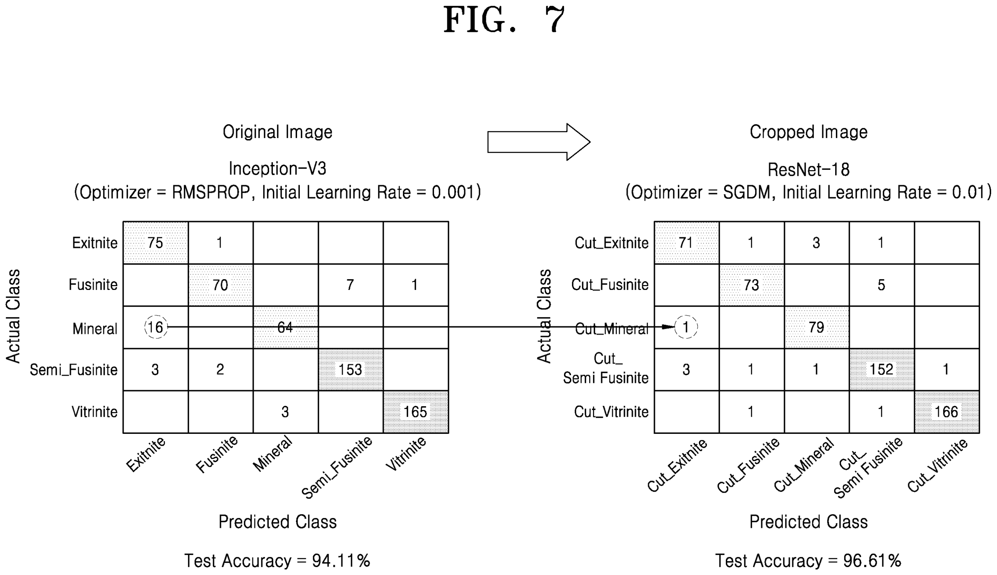

As shown in FIG. 6, it was confirmed that the validation accuracy and test accuracy of the three types of deep learning networks improved to approximately 96%. In particular, as shown in FIG. 7, it was confirmed that 16 images that incorrectly classified Mineral as Exinite when inputting the entire specimen image could be effectively classified when extracting the central region.

FIG. 8 is an image showing an attention region that an Al model sets for analysis on the specimen image in step (c) of the material analysis method according to an embodiment of the present invention utilizing a Class Activation Map (CAM).

It was found that all of Exinite, Fusinite, Semi-fusinite, and Mineral except Vitrinite analyzed a region corresponding to the central region by artificial intelligence. Therefore, when analyzing by extracting an attention region, which is a region corresponding to the central region on the specimen image, in step (c) of the material analysis method according to an embodiment of the present invention to obtain an analytical image, test precision of approximately 96% can be secured.

FIG. 9 is a table measuring and comparing validation accuracy and test accuracy of ResNet-50 and MobileNet V2 deep learning networks, which are deep learning networks of the Al model of the material analysis method according to an embodiment of the present invention.

When performing step (c) of detecting noise, that is, epoxy images, by applying the above two types of deep learning networks to the noise removal Al model of the material analysis method according to an embodiment of the present invention, epoxy could be distinguished with very high accuracy, with 100% validation accuracy and 99.86% test accuracy, respectively.

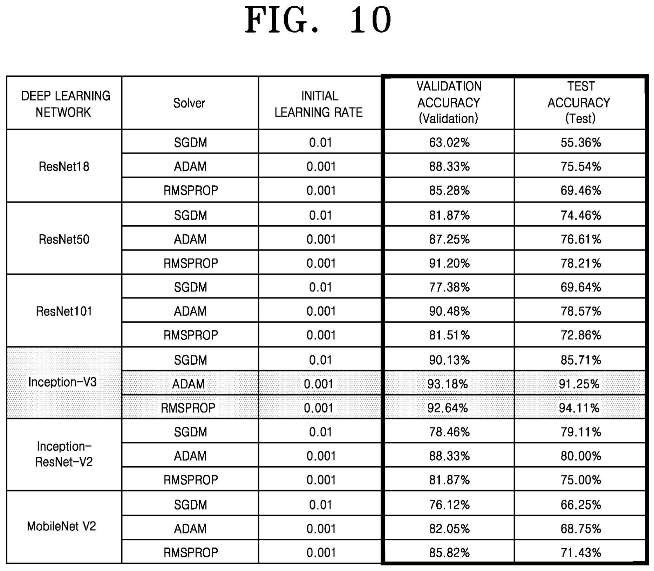

FIG. 10 is a table measuring and comparing validation accuracy and test accuracy of Inception V3 and other types of deep learning networks, which are deep learning networks of the Al model of the material analysis method according to an embodiment of the present invention.

As shown in FIG. 10, validation accuracy and test accuracy were measured by applying an initial learning rate of 0.01 or 0.001 to three types of solvers for each of the deep learning networks of ResNet, Inception-ResNet, MobileNet, and Inception V3 applied to the present invention. As a result of measurement, it was confirmed that Inception V3 showed the highest validation accuracy (93.18%, 92.64%) and the highest test accuracy (91.25%, 94.11%) at an initial learning rate of 0.001.

That is, as a result of repeated experiments, the applicant confirmed that when applying an initial learning rate of 0.001 to the ADAM or RMSPROP solver in the combination of a ResNet Al model and an Inception V3 Al model, or the combination of a MobileNet Al model and an Inception V3 Al model, coal Maceral microstructure can be classified with the highest accuracy.

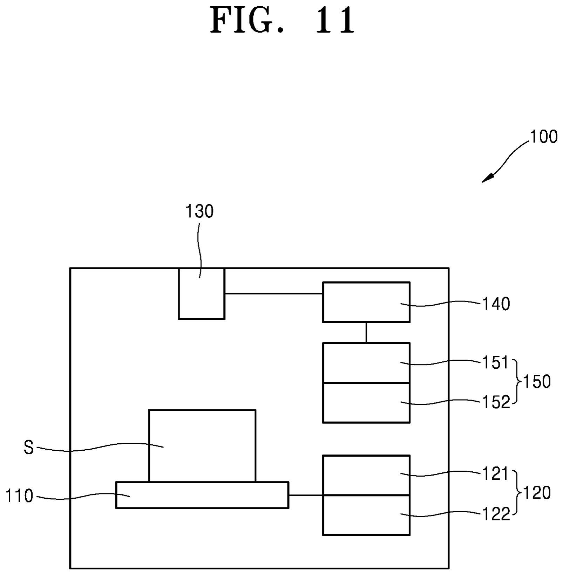

Hereinafter, a material analysis apparatus 100 according to an embodiment of the present invention will be described with reference to FIGS. 11 to 12.

FIG. 11 is a conceptual diagram schematically showing a material analysis apparatus 100 according to an embodiment of the present invention. As shown in FIG. 11, the material analysis apparatus 100 according to an embodiment of the present invention may include a stage 110, a stage moving device 120, an image capturing device 130, and a microstructure classification device 140.

More specifically, the stage moving device 120 may include a stage front-rear moving device 121 capable of moving the stage 110 in a front-rear direction, and a stage left-right moving device 122 capable of moving the stage 110 in a left-right direction.

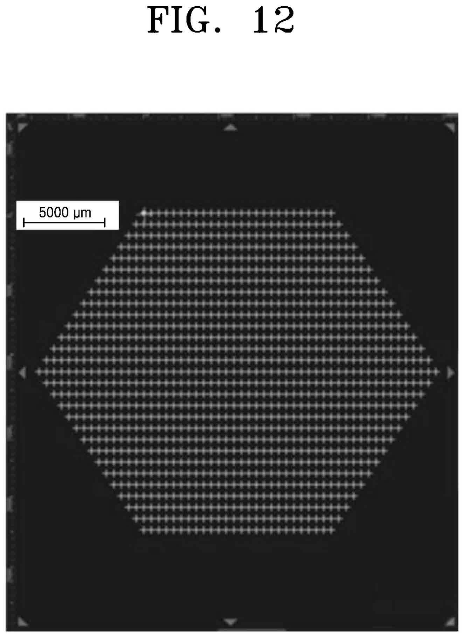

In addition, the image capturing device 130 may photograph an image of the specimen by setting an inspection region on one surface of the specimen, as shown in FIG. 12. At this time, the inspection region may be a region in which a plurality of inspection points are set to form an overall hexagonal shape. Such an inspection region may be a region in which a plurality of inspection points are set to form an overall hexagonal shape as shown in FIG. 12.

Therefore, the stage moving device 120 may, for example, move the stage 110 so that the image capturing device 130 is positioned at an inspection point of any one vertex among the vertices of a hexagon, and then move the stage 110 forward-backward or left-right so that the image capturing device 130 is positioned at an inspection point of another adjacent vertex.

Conventionally, inspection regions were generally set so that inspection points form an overall quadrilateral shape, but in the present invention, by setting the above-mentioned hexagonal inspection region, characteristics of the entire circular cross-section could be sufficiently reflected, and when setting an inspection region to form a polygonal shape of hexagon or more, there was a problem that the time required for inspection was lengthened due to an excessively large number of inspection points, so the most efficient analysis could proceed when a hexagonal inspection region was set.

In addition, the material analysis apparatus 100 according to an embodiment of the present invention may include a deep learning unit 150. At this time, the deep learning unit 150 may include a noise removal deep learning unit 151 that learns and classifies noise microstructure patterns so as to distinguish and classify noise appearing in the specimen, and a microstructure classification deep learning unit 152 that learns and classifies target microstructure patterns so as to distinguish and classify microstructure patterns appearing in the specimen.

Therefore, images of inspection points of the specimen photographed by the image capturing device 130 can be classified into Vitrinite, Exinite, Fusinite, Semi-fusinite, and Mineral using an Al model trained using deep learning.

The present invention has been described with reference to embodiments shown in the drawings, but this is merely exemplary, and those skilled in the art will understand that various modifications and equivalent other embodiments are possible therefrom. Therefore, the true technical protection scope of the present invention should be determined by the technical spirit of the appended claims.

## Claims

1. A material analysis method comprising:
(a) preparing a specimen for analyzing a microstructure;
(b) acquiring a plurality of specimen images by photographing an inspection region set on one surface of the specimen;
(c) preprocessing the plurality of specimen images to obtain a plurality of analytical images; and
(d) analyzing the plurality of analytical images using a trained microstructure classification artificial intelligence (AI) model to classify into at least one target microstructure pattern.

2. The material analysis method of claim 1, wherein the step (a) comprises:
(a-1) crushing a material into particles having a predetermined size; and
(a-2) mixing the particles with a binder to fabricate the specimen.

3. The material analysis method of claim 2, wherein the material is coal, and
wherein the binder is epoxy.

4. The material analysis method of claim 1, wherein the step (c) comprises:
extracting an attention region that an Al model sets for analysis on the specimen image utilizing a Class Activation Map (CAM) to obtain an analytical image.

5. The material analysis method of claim 4, wherein the attention region is a region corresponding to a central region on the specimen image.

6. The material analysis method of claim 1, wherein the step (c) comprises:
analyzing a plurality of specimen images in which a microstructure pattern of the specimen is not labeled using a trained noise removal artificial intelligence (AI) model to remove analytical images classified as a preset noise microstructure pattern.

7. The material analysis method of claim 6, wherein the noise microstructure pattern is an image in which the microstructure pattern of the specimen is classified as a binder by the noise removal Al model.

8. The material analysis method of claim 6, wherein the noise removal Al model is an Al model using a Residual neural network (ResNet) or MobileNet based deep learning network.

9. The material analysis method of claim 1, wherein in the step (d), the microstructure classification Al model is an Al model using an Inception based deep learning network.

10. The material analysis method of claim 1, further comprising, before the step (c), (f) training a noise removal Al model or a microstructure classification Al model with training images in which a microstructure pattern of an arbitrary specimen is labeled.

11. The material analysis method of claim 1, wherein in the step (d), the target microstructure pattern is an image in which the microstructure pattern of the specimen is classified by the second Al model into at least one of Vitrinite, Exinite, Fusinite, Semi-fusinite, Mineral, and combinations thereof.

12. The material analysis method of claim 1, further comprising, after the step (d),
(e) calculating a proportion of the classified microstructure patterns of the specimen.
